(19) Europäisches Patentamt

European Patent Office

Office européen des brevets

(11) **EP 4 751 700 A1**

(12) **EUROPEAN PATENT APPLICATION**

(43) Date of publication:
**03.06.2026 Bulletin 2026/23**

(21) Application number: **24216016.6**

(22) Date of filing: **28.11.2024**

(51) International Patent Classification (IPC):
***A61K 8/02*** *(2006.01)*     ***A61K 8/25*** *(2006.01)*
***A61Q 11/00*** *(2006.01)*

(52) Cooperative Patent Classification (CPC):
**A61Q 11/00; A61K 8/0241; A61K 8/25;**
A61K 2800/412

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(71) Applicant: **Haleon UK IP Limited
Weybridge, Surrey KT13 0NY (GB)**

(72) Inventor: **The designation of the inventor has not
yet been filed**

(74) Representative: **Haleon Patent Department
Building 5, First Floor
The Heights
Weybridge, Surrey KT13 0NY (GB)**

(54) **COMPOSITION**

(57)     A dentifrice composition comprises a tubule blocking agent including a bioactive glass, wherein the tubule blocking agent has a particle size less than 10 μm.

**EP 4 751 700 A1**

**Description**

**BACKGROUND**

Oral care products have been used to treat oral health issues for centuries. One such oral health concern is hypersensitivity.

This is a well-known phenomenon; people suffering from dentinal (hyper) sensitivity experience a painful reaction when their teeth are exposed to cold, hot, sweet, drying, tactile and similar stimuli, e.g. when eating ice-cream or brushing their teeth. The painful stimulus is generally attributed to rapid fluid movements within the dentinal tubules which run through the thickness of the dentine to the nerve terminals in the pre- dentine.

Dentine contains fluid-filled tubules, which carry stimuli from the outer enamel to the nerves in the pulp cavity through the movement of the fluid. Dentine hypersensitivity arises from the exposure of the tubules to the oral environment, through enamel loss or gum recession. This can result in excessive fluid movement, causing discomfort

Bioactive glasses such as NovaMin® can be incorporated into at-home dentifrices to occlude patent tubules with a hydroxyapatite-like (HA-like) layer and reduce symptoms. This has been found to break down to form protective Hydroxyapatite-like layer, reoccluding patent tubules

Whilst such agents have been found to be useful in addressing dentine hypersensitivity there is a continual need for improvement in the treatment of dentine hypersensitivity in a user.

Accordingly, there is a desire for improved oral care compositions, that are alleviating dentinal (hyper) sensitivity for a user.

**STATEMENT OF INVENTION**

According to the present invention there is provided a dentifrice composition comprising a tubule blocking agent including a bioactive glass, wherein the tubule blocking agent has a particle size less than 10 $\mu$m.

The invention also comprises the use of the composition in combination with a toothbrush to clean teeth.

The invention also features a method for tooth whitening including applying the tooth composition of the present invention to at least one tooth.

As used herein the term "dentifrice" includes any semi-solid preparation in the form of a paste, cream or gel for use in cleaning or treating all, or a portion of, the oral cavity of an individual.

The present inventors have discovered that the dentifrice of the invention provides excellent sensitivity protection; allowing users to whiten teeth/dentures with confidence.

In more detail it has been found that the dentifrice of the invention, by reducing the particle size of the tubule blocking agent increased the reactivity of same. This is associated with greater reactivity and faster apatite formation (in, for example, the first 24 hours).

It had no significant effect on the mechanical or crystallographic properties of the HA-like surface layer formed by the dentifrice (even after, for example, 5 days of development). This has been found to be associated with no change in hardness or reduced modulus and no change in crystallite size.

It has been found to result in greater tubule occlusion and greater penetration of material deeper into the tubules (even after, for example, only 24 and 48 hours).

**DETAILED DESCRIPTION OF THE INVENTION**

Typically, novamin with a particle size in the order of 15 $\mu$m has been used in commercial dentifrices (Sensodyne repair and protect range as sold by Haleon) to relieve the symptoms of hypersensitivity in users.

The applicants have surprisingly found that reducing the particle size of the bioglass can provide significantly improved properties.

*Tubule Blocking Agent Particle Size*

The tubule blocking agent has a particle size less than about 10 $\mu$m.

Preferably the tubule blocking agent has a particle size less than about 9 $\mu$m, more preferably less than about 8 $\mu$m, more preferably less than about than 7 $\mu$m, more preferably less than about 6 $\mu$m, more preferably less than about 5 $\mu$m, more preferably less than about 4 $\mu$m, more preferably less than about 3 $\mu$m, more preferably less than about 2.5 $\mu$m, more preferably less than about 2.25 $\mu$m, more preferably less than about 2.1 $\mu$m, more preferably less than about 2 $\mu$m.

Alternatively and / or additionally the tubule blocking agent preferably has an average particle size in the range of about 0.01 to about 10 $\mu$m, more preferably in the range of about 0.01 to about 9 $\mu$m, more preferably in the range of about 0.01 to about 8 $\mu$m, more preferably in the range of about 0.01 to about 7 $\mu$m, more preferably in the range of about 0.01 to

about 6 $\mu$m, more preferably in the range of about 0.01 to about 5 $\mu$m, more preferably in the range of about 0.01 to about 4 $\mu$m, more preferably in the range of about 0.01 to about 3 $\mu$m, more preferably in the range of about 0.01 to about 2.5 $\mu$m, more preferably in the range of about 0.01 to about 2.25 $\mu$m, more preferably in the range of about 0.01 to about 2.1 $\mu$m, more preferably in the range of about 0.01 to about 2 $\mu$m. In each of these ranges the lower amount (about 0.01 $\mu$m) may be replaced by about 0.1 $\mu$m, about 0.2 $\mu$m, about 0.3 $\mu$m, about 0.4 $\mu$m, about 0.5 $\mu$m, about 0.6 $\mu$m, about 0.7 $\mu$m, about 0.8 $\mu$m, about 0.9 $\mu$m, about 1.0 $\mu$m, 1.1 $\mu$m, about 1.2 $\mu$m, about 1.3 $\mu$m, about 1.4 $\mu$m, about 1.5 $\mu$m, about 1.6 $\mu$m, about 1.7 $\mu$m, about 1.8 $\mu$m, or about 1.9 $\mu$m (wherein the tubule blocking agent has a particle size less than 10 $\mu$m).

Alternatively and / or additionally the tubule blocking agent preferably has a particle size in the range of about 0.01 to about 10 $\mu$m, more preferably in the range of about 0.01 to about 9 $\mu$m, more preferably in the range of about 0.01 to about 8 $\mu$m, more preferably in the range of about 0.01 to about 7 $\mu$m, more preferably in the range of about 0.01 to about 6 $\mu$m, more preferably in the range of about 0.01 to about 5 $\mu$m, more preferably in the range of about 0.01 to about 4 $\mu$m, more preferably in the range of about 0.01 to about 3 $\mu$m, more preferably in the range of about 0.01 to about 2.5 $\mu$m, more preferably in the range of about 0.01 to about 2.25 $\mu$m, more preferably in the range of about 0.01 to about 2.1 $\mu$m, more preferably in the range of about 0.01 to about 2 $\mu$m. In each of these ranges the lower amount (about 0.01 $\mu$m) may be replaced by about 0.1 $\mu$m, about 0.2 $\mu$m, about 0.3 $\mu$m, about 0.4 $\mu$m, about 0.5 $\mu$m, about 0.6 $\mu$m, about 0.7 $\mu$m, about 0.8 $\mu$m, about 0.9 $\mu$m, about 1.0 $\mu$m, 1.1 $\mu$m, about 1.2 $\mu$m, about 1.3 $\mu$m, about 1.4 $\mu$m, about 1.5 $\mu$m, about 1.6 $\mu$m, about 1.7 $\mu$m, about 1.8 $\mu$m, or about 1.9 $\mu$m.

Particle size measurements carried out by laser diffraction according to standard techniques.

*Amount of Tubule Blocking Agent*

Preferably the tubule blocking agent is present in an amount of 0.1 to 10 percent by weight of the composition. The tubule blocking agent may be present in an amount of 1 to 9 percent, 2 to 8 percent, 3 to 7 percent, 4 to 6 percent, about 5 percent, by weight of the composition.

The tubule blocking agent may comprise a single material, or the tubule blocking agent may comprise two more distinct and discrete materials

*Nature of Tubule Blocking Agent*

The tubule blocking agent includes a bioactive glass.

Suitably the bioactive glass consists of 45 percent by weight silicon dioxide, 24.5 percent by weight sodium oxide, 6 percent by weight phosphorus oxide, and 24.5 percent by weight calcium oxide. One such bioactive glass is available commercially under the trade name, NOVAMIN, also known as 45S5 BIOGLASS.

Another alternate tubule blocking agent may be present in addition to the bioactive glass.

The alternate tubule blocking agent is preferably selected from the list comprising silica, colloidal silica, nano zinc oxide, sub-micron alumina and sub-micron polymer beads, or mixtures thereof.

Where present the alternate tubule blocking agent preferably has a similar size to the bioactive glass.

Preferably the bioactive glass is the sole tubule blocking agent in the composition.

*Anti-sensitivity*

An additional desensitizing agent, including a tubule blocking agent or a nerve desensitizing agent and mixtures thereof, for example as described in WO 02/15809 (Block) maybe included in the composition according to the invention.

Preferably an additional desensitizing agent is present in an amount of between 0.1 and 10 percent by weight of the composition.

The desensitizing agent may comprise a strontium salt such as strontium chloride, strontium acetate or strontium nitrate or a potassium salt such as potassium citrate, potassium chloride, potassium bicarbonate, potassium gluconate and especially potassium nitrate.

*Diluent*

Preferably the dentifrice comprises a single phase non-aqueous composition comprising at least one solvent.

Preferably the at least one solvent comprises glycerol, polyethylene glycol (PEG) or a mixture thereof.

Glycerine is also known as glycerol and glycerin. The terms may be used interchangeably in this specification.

It is well known that commercially available glycerine may contain between 0.1-2.0 percent by weight of water which is in association with the glycerine. Typically, this amount is < 0.5 percent and for example between 0.1 - 0.5 percent by weight of the glycerine. This small amount of water is bound to the glycerine and is therefore not available to the other ingredients. The skilled person would still consider a composition containing glycerine as being non-aqueous.

Preferably the at least one solvent comprises up to 85 percent by weight of the final composition. Preferably the amount of glycerol is up to 10 percent by weight of the final composition; most preferably about 5 percent by weight. Preferably the amount of polyethylene glycol (PEG) is up to 75 percent by weight of the final composition; most preferably about 60 to 70 percent by weight.

*Gel*

The compositions according to the invention may comprise a supporting (or gelling) polymer to solidify the solvent and actives.

The skilled person will be aware of many different types of supporting polymer for dentifrice use. Two non-limiting examples include a copolymer of a methyl vinyl ether and a maleic anhydride, such as Gantrez(R), and polyacrylic acid homopolymers, often referred to as Carbomers.

Suitably for the present invention the supporting (or gelling) polymer comprises a carboxyvinyl polymer such as a carbomer.

A carbomer comprises synthetic high molecular-weight cross-linked polymers of acrylic acid. The polymer chains formed of repeating units of acrylic acid may be cross-linked with, for example: allyl sucrose to provide a carbomer available commercially in one form as Carbopol™ 934; ethers of pentaerythritol to provide a carbomer available commercially in one form as Carbopol™ 974; or with divinyl glycol, available commercially in one form as Noveon™ AA-1. Carbopol™ polymers are manufactured by B.F. Goodrich Company.

In one embodiment the carboxyvinyl polymer comprises Carbopol™ 974.

The carboxyvinyl polymer may be present in the range of from about 0.1 to about 7.5 percent by weight of the non-aqueous composition. In one embodiment the carboxyvinyl polymer is present in an amount from about 0.2 to about 1.0 percent by weight of the composition.

*Abrasive*

An abrasive may be required to aid the removal of detritus from the teeth, dentures and / or remainder of the oral cavity being cleaned. Clearly it will be appreciated that a degree of abrasiveness is required / advantageous in the removal of said detritus., However, it will also be appreciated that excessive abrasiveness can be equally detrimental: excessive wear on dental materials can be problematic. As such it will be appreciated that the use of an abrasive is a compromise of achieving adequate detritus removal without causing excessive /undesired wear on dental materials.

Suitable abrasives for use in the dentifrice composition include, for example, amorphous, gelled, precipitated or fumed silica, zinc orthophosphate, sodium bicarbonate (baking soda), plastic particles, alumina, hydrated alumina, calcium carbonate, calcium pyrophosphate, insoluble metaphosphates or mixtures thereof.

The silica abrasive may be a natural amorphous silica, for instance diatomaceous earth; or a synthetic amorphous silica such as a precipitated silica. By way of example, silica abrasives include those marketed under the following trade names Zeodent, Sident, Sorbosil or Tixosil by Huber, Degussa, Ineos and Rhodia respectively.

The silica abrasive may be a spherical, anhydrous, amorphous, silica gel particles having a pore volume of less than 0.1ml/g (as described in co-pending European Application Number 18782931.2). This abrasive preferably comprises non-fused spherical, anhydrous, amorphous, silica gel particles obtainable by a process that involves precipitation followed by calcination to remove water to produce a dense particulate silica material, and wherein said silica gel particles comprise:

a. a pore volume from 0.03 ml/g to less than 0.1 ml/g;
b. a mean particle size from 1 micron to 10 micron;
c. a BET surface area of 50 $m^2$/g or less;
d. an oil absorption capacity of 20 to 50 mL/100 g; and
e. a water content of less than 0.2 weight percent.

Such silica gel particles have been found to effectively clean, polish and remove stains from the surface of teeth or dentures without a high degree of abrasion thereby reducing scratching and damage to the tooth or denture surface. Such compositions thereby provide superior cleaning, polishing, gentle stain removal and whitening of tooth surfaces or dentures.

Silica gel particles for use in the invention are available commercially from Asahi Glass SI-Tech. Co., Ltd., 13-1, Kitaminatomachi"Wakamatsu-ku, Fukuoka, 808-0027, Japan. and are sold under the brand name Sunspherc(a), for example Sunsphcre NP-30 and Sunsphcrc NP-100. A CAS1 for some types of silica gel particles of use in the invention is 7631-86-9.

Sunsphere(a) silica gel particles are derived from sodium silicate and contain no crystalline silica. They consist of spherical particles of almost identical size which do not cohere, and which provide a smooth texture.

Similar silica gel particles are available from Madhu Silica PVT. LTD under the brand name ( as an example) of MFIL-GS.

Most preferably where an abrasive silica is used it is desirable that the stability of hydrogen peroxide in presence of silica is preserved. It has been observed that spherical, anhydrous, amorphous, silica gel shows high preservation of hydrogen peroxide. As such these are highly preferred to be used as an abrasive silica.

Suitably an abrasive is present in an amount up to 5 percent by weight of the total composition, for example from 0.1 to 4 percent by weight, for example from 0.1 to 3 percent, for example from 0.1 to 2 percent, for example from 0.1 to 1.5 percent, for example from 0.2 to 1 percent, for example from 0.25 to 1 percent by weight of the total composition.

Generally, an amount of abrasive suitable for use in the dentifrice composition of the present invention will be empirically determined to provide an acceptable level of cleaning and polishing, in accordance with the techniques well known in the art.

*Fluoride*

The dentifrice compositions of the present invention preferably comprise a source of fluoride as required. Non-limiting examples of fluoride include stannous fluoride, sodium fluoride, sodium monofluorophosphate, potassium fluoride, ammonium fluoride, bis-(hydroxyethyl) amino-propyl-N-hydroxyethyloctadecylamine-dihydrofluoride and mixtures thereof. Preferably the fluoride required in the formula will be provided by stannous fluoride ($SnF_2$) and / or sodium fluoride (NaF).

Suitably the composition comprises between 0.05 percent and 0.5 percent by weight of fluoride source. Preferably this equates to from 450ppm of fluoride to 1500ppm of fluoride. A preferred amount of fluoride is about 900ppm, or about 1150ppm or about 1450ppm.

The skilled person will be aware that the amount of fluoride present in commercial oral care compositions is dependent on and limited by national and regional regulation and health and safety law.

*Surfactant*

The dentifrice composition according to the invention may comprise at least one surfactant. The at least one surfactant may comprise a single discrete surfactant. Alternatively, the at least one surfactant may comprise a mixture of a first surfactant and a second surfactant. Alternatively, the at least one surfactant may comprise a mixture of a first surfactant, a second surfactant and a third and/or further surfactants.

A suitable surfactant type for use in the compositions according to the invention belongs to the class of compounds known as betaines. Structurally, betaine compounds contain an anionic functional group such as a carboxylate functional group and a cationic functional group such as quaternary nitrogen functional group separated by a methylene moiety. They include n-alkyl betaines such as cetyl betaine and behenyl betaine, and n-alkylamido betaines such as cocoamidopropyl betaine.

In one embodiment the betaine is cocoamidopropyl betaine, commercially available under the trade name Tego Betain(R).

Suitably the betaine is present in an amount ranging from about 0.05 to about 4 percent by weight of composition, for example from about 0.2 to about 2.0 percent, more preferably between about 0.3 to about 0.6 percent by weight of the non-aqueous composition. Another surfactant for use in the dentifrice compositions according to the invention is selected from a taurate based surfactant. Taurate surfactants useful in the present invention are salts of fatty acid amides of N-methyl taurine. They conform generally to the structural formula:

$$RC(O)N(CH_3)CH_2CH_2SO_3$$

Where RC(O)- represents a fatty acid radical and M represents sodium, potassium, ammonium or triethanolamine. Fatty acids having carbon chain lengths of from 10 to 20, including those derived from coconut, palm and tall oil are used. In one embodiment the fatty acid is derived from coconut. In one embodiment, sodium salts are used.

In one embodiment the taurate is sodium methyl cocyl taurate. This taurate surfactant is sold under the trademark by Adinol(R) CT by Croda.

The taurate surfactant may be present in an amount from about 0.1 to about 10 percent of the anhydrous dentifrice composition. In one embodiment the taurate surfactant is present in an amount from about 0.1 to about 5 percent by weight of the non-aqueous composition. In one embodiment the taurate surfactant is present in an amount from about 0.5 to about 2.0 percent by weight of the non-aqueous composition.

Another class of surfactants suitable for the present invention are alkyl sulphate surfactants of the following structural formula:

$$R^1OSO_3$$

$R^1$ represents a fatty alcohol moiety and M represents sodium, potassium, ammonium or triethanolamine. Fatty alcohols having carbon chain lengths of from about 10 to about 20, including those derived from coconut, palm oil and tall oil. In one embodiment, the fatty alcohol is lauryl alcohol. In one embodiment, a sodium salt is used.

In one embodiment the alkyl sulphate is sodium lauryl sulphate.

The alkyl sulphate surfactant may be present in an amount from about 0.1 to about 10 percent of the nonaqueous composition. In one embodiment the alkyl sulphate surfactant may be present in an amount from about 0.1 to about 5 percent by weight of the non-aqueous dentifrice composition.

In one embodiment the alkyl sulphate surfactant is present in an amount from about 0.5 to about 2.0 percent by weight of the non-aqueous composition. In certain embodiments, the at least one surfactant comprises a first surfactant which is a C10-20 alkyl sulphate surfactant, and a second surfactant which consists of a betaine. Preferably wherein the first surfactant is sodium lauryl sulphate (SLS), and the second surfactant is cocamidopropyl betaine.

Preferably the ratio of SLS and cocamidopropyl betaine is between 2:1 and 4:1 by weight. Most preferably the ratio of the SLS to cocamidoproyl betaine is about 3:1.

In a particularly preferred embodiment, SLS comprises between 1.0 and 1.4 percent by weight and the cocamidopropyl betaine comprises between 0.3 and 0.5 percent by weight of the dentifrice composition.

*Anti-Stain*

The composition preferably comprises an anti-staining agent.

Preferably, where present, the anti-staining agent is present in an amount of up to 10 percent of the weight of the composition. Preferably the anti-staining agent is present in an amount of about 5 percent weight of the composition.

A preferred example of an ant-staining agent is anhydrous sodium tripolyphosphate.

*Other*

Compositions of the present invention may contain additional formulation agents such as flavouring agents, sweetening agents, opacifying or colouring agents and preservatives, selected from those conventionally used in an oral hygiene composition art for such purposes.

In general, the optional agents may be used in a minor amount or proportion of the overall formulation. By way of example, such components are usually present in from about 0.001 to about 5 percent by weight of the non-aqueous composition. In total such components may range from 0.1 to 25 percent by weight of the composition.

The dentifrice composition typically has a viscosity suitable for application to the oral cavity. The viscosity will vary depending on the type of dentifrice composition made and the ultimate use thereof. One of skill in the art can readily prepare compositions with suitable viscosities for use in the oral cavity from the teachings provided herein. Suitably a composition according to the invention may further comprise an inorganic thickening agent such as a thickening silica. Suitably, the thickening agent is a thickening silica, for example, a colloidal hydrated silica, available commercially for example as Sident 22S or Syloid 244FP.

In one embodiment the thickening silica is present in the range of from about 0 to about 15 percent, suitably from about 5.0 to about 15.0 percent by weight of the dentifrice composition.

The compositions according to the present invention may be prepared by admixing the ingredients in the appropriate relative amounts in any order that is convenient.

A dentifrice composition according to the present invention may be mildly acidic, neutral or mildly alkaline i.e. has a slurry pH in the range from 6.0 to 7.5, for example from pH 6.1 to 7.4, 6.2 to 7.3, or 6.2 to 7.2. In one embodiment the composition has a pH of about 6.2. In a further embodiment the composition has a pH of about 7.0. The pH referred to is that measured when the dentifrice composition is slurried with water in a 1:3 weight ratio of the composition to water. Suitably the slurry is prepared by slurring the dentifrice composition with water in a weight ratio of one part dentifrice composition and three parts distilled water. The pH is determined using a standard pH meter.

The dentifrice compositions of the present invention may advantageously be nonaqueous dentifrice compositions. As used herein, the term "non-aqueous" means anhydrous or substantially free of water. The individual components of the nonaqueous composition may contain limited amounts of water as long as the overall composition remains substantially free of water.

***Examples***

• ***Tubule Occlusion***

*Methodology*

Tubule Occlusion was tested as follows.

7 treatment groups were used.

In each Sensodyne® Repair and Protect was used as the base toothpaste: This contained 5 wt% of different particle sizes of NovaMin® as follows:

| Sensodyne Repair and Protect | |
|---|---|
| **Ingredient** | **% weight** |
| Glycerol | 60 |
| PEG 400 | 20 |
| Thickening silica | 10 |
| Novamin | 5 |
| Carbomer | 0.84 |
| Surfactant | 2.4 |
| Minor ingredients | to 100 |

- 15 $\mu$m (to represent the current commercial composition),
- 2, 4, 7, 10 &15 $\mu$m)
- NovaMin-free Sensodyne base (0%)
- Artificial Saliva (AS) only (0.019 g/L $MgCl_2$, 0.147 g/L $CaCl_2$, 0.544 g/L $KH_2PO_4$, 4.766 g/L HEPES, 1.193 g/L KCl, 0.241 g/L NH4Cl; this was buffered to pH 7 with 1M Potassium Hydroxide)

This was brushed onto human dentine, as follows:-

- 20 seconds per sample, twice per day; 2mg paste + 0.5ml AS
- Stored in AS after each brush
- Brushed for 1 day, a section of sample removed, rest brushed for another day (24hrs + 48hrs)

One 1 mm sample was trimmed from each block after the first 2 treatments (establishing a 24-hour sample), and another was taken after a further two, forming the 48-hour samples. For each treatment, a soft manual toothbrush was fitted to a reciprocating brushing rig, whose sample plate was rotating in a 5 mm radius circular motion [Phoenix Tribology, Kingsclere, UK].

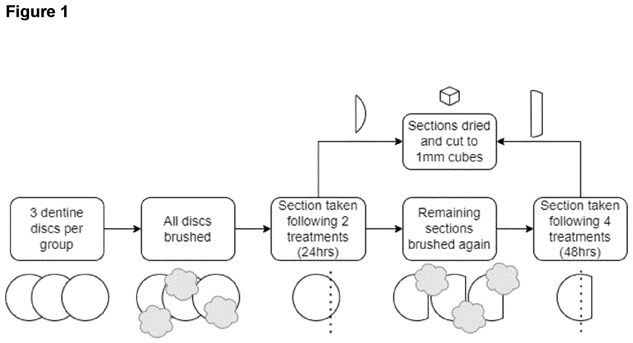

This portion of the treatment method is shown in Figure 1.

Each 1 mm block was embedded into resin and mounted on to SEM pins. No heavy metal staining or tissue demineralisation took place, and samples were aligned such that the treated sample surface was facing upwards from the pin. The samples were imaged using a 3View® 2XP [Gatan UK, Abingdon, UK] attachment on an FEI Quanta 250 field emission gun SEM [Thermo Fisher Scientific, Eindhoven, Netherlands]. A vacuum level of 20 Pa was used, and an accelerating voltage of 2.5 kV. The chosen image size was 4500 x 4500 pixels, with a 40 nm pixel size and a dwell time of 5 $\mu$s, resulting in an FOV of 180 x 180 $\mu$m. Each slice was 100 nm thick, and a minimum of 500 slices were taken from each block, resulting in a minimum stack depth of 35 $\mu$m.

100 tubules selected at random for analysis (50 per sample, n=2 per group per timepoint)

Occlusion on surface was recorded, as well as the depth of any penetrating material.

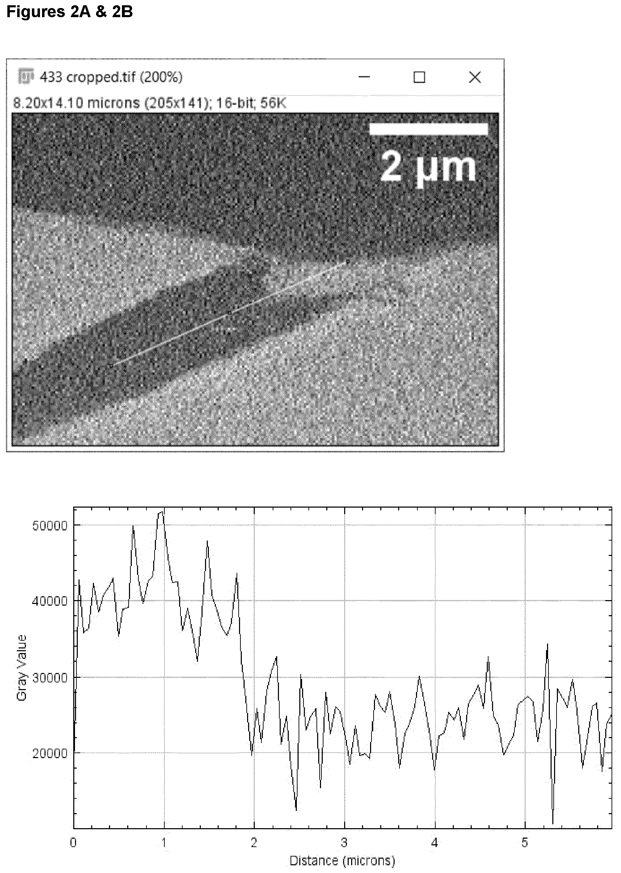

Individual tubules resliced into cross-section view and the grey value along tubule length recorded, significant drop identified as end of occlusive plug are illustrated pictorially in Figures 2A & 2B.

*Surface Results*

Table 1 shows the percentage of tubule occluded for each group after 24 and 48 hours.

_Table 1_

| Treatment Group | Tubules occluded after: | |
| --- | --- | --- |
| | _24 hours (%)_ | _48 hours (%)_ |
| AS only | 47 | 44 |
| NovaMin®-free control | 61 | 65 |
| 2µm NovaMin® | 91 | 93 |
| 4µm NovaMin® | 88 | _90_ |
| 7µm NovaMin® | 85 | 88 |
| 10µm NovaMin® | 87 | 87 |
| 15µm NovaMin® | 84 | 87 |

All NovaMin® groups showed more occlusion than either the AS or NovaMin®-free control.

All groups, with the exception of the AS control, showed more occlusion after 48 hours than 24. Also, there is a trend showing greater occlusion capabilities in the smaller particle sizes, with the 2 and 4 µm NovaMin® showing more occlusion after 24 hours than the 10 or 15 µm NovaMin® did after 48 hours.

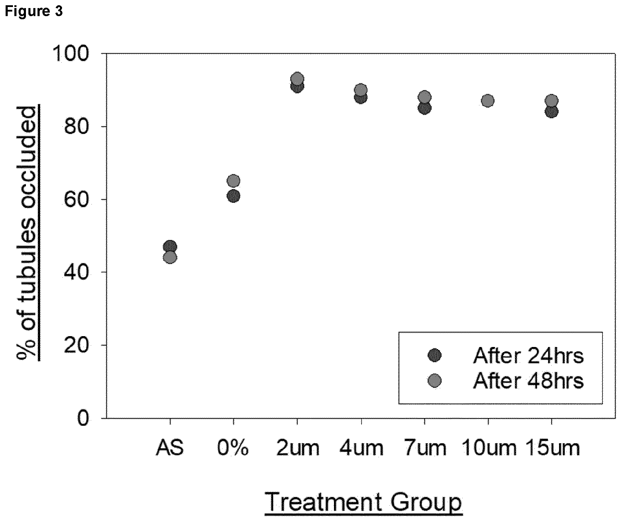

Figure 3 shows the average percentage of occlusion for each group after 24 and 48 hours.

_Depth Results_

Again, all NovaMin® groups showed deeper occlusion penetration than either control.

All groups showed deeper average occlusion depths after 48 hours than 24. A similar trend to overall occlusion was also observed, with the 2 µm particle size showing the highest occlusion depths, and the 15 µm the lowest of all the tested NovaMin® groups. Due to the large standard deviations observed, little statistical significance was found between groups. After 24 hours, only the 2 µm NovaMin® group was significantly higher than the AS control ($p = 0.005$). After 48 hours, only the 2 µm NovaMin® group was significantly higher than both the AS and NovaMin®-free controls ($p < 0.05$).

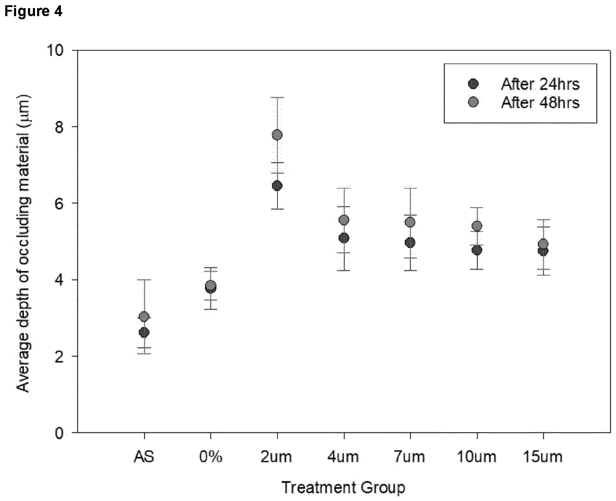

Figure 4 shows the average depth of occlusion for each group after 24 and 48 hours.

### • Effect of particle size on apatite formation

Nanoindentation was used to determine the effect of the particle size on the mechanical properties of the surface layer.

Contrary to other indentation techniques, such as Vickers Hardness, nanoindentation generates full depth vs load curves for each indent, from which the hardness and reduced modulus can be calculated. Investigating these parameters is key to understanding the protective nature of the generated surface layer. The oral environment regularly undergoes both abrasive and acidic challenges during daily life, so an active ingredient must be able to form surface layers with mechanical properties capable of withstanding such challenges. Another benefit to nanoindentation over other techniques is the additional sensitivity to depth.

The interaction volume under the indent scales proportionally with the size of the indenter tip, meaning the indentations can be tailored to investigate a much smaller and more specific volume of material. This was beneficial in this scenario as it meant the HA-like layer could be investigated on its own, without measuring the properties of the dentine substrate underneath, which would produce a composite result.

The surface layers were investigated using Glancing Angle X-Ray Diffraction (GA-XRD). X-rays are accelerated towards the sample surface through a range of $2\theta$ angles, which are then diffracted by sample atoms or planes. This creates a spectrum, from which crystallographic information about a sample can be determined, namely the size of individual crystallites. Understanding the crystal structure is important, as this can also affect the protectiveness offered, with larger crystallites showing greater erosive and abrasive resistance.

Both of the above tests were performed on the surface layer that had been generated on the surface of dentine samples over the course of several days. However, it was also important to investigate the short-term reaction rate of the BAG powder, to determine the effect of decreasing the particle size. XRD analysis of the BAG powder was performed after a series of reactions with AS ranging from 1 to 36 hours. As well as being able to quantify crystallite size, as with the GA-XRD, the main comparison for this investigation was qualitatively comparing the spectra. This was done by observing the peaks corresponding to apatite material develop and become more pronounced over time, and directly comparing between different BAG particle sizes. Thus, an approximate reaction rate can be determined and compared between groups. The crystallite size was investigated with both GA- and XRD, as it was proposed that reactions progressing at different rates would result in different crystallite sizes. Also, more smaller particles would have been included in the formulation for the same wt.% concentration, potentially providing additional nucleation sites for HA-like material to form.

*Methodology*

*Dentine samples*

Samples of bovine dentine were supplied by Modus Laboratories [Plymouth, UK], under ethical approval from the University of Southampton (ERGO approval number 53503). 28 dentine squares with an approximate width of 6 mm and thickness of 1 mm were manufactured from the facial side of bovine incisors. The teeth were sectioned and polished using 3 $\mu$m and 1 $\mu$m diamond suspensions and etched with 1 wt.% citric acid solution to remove the smear layer. Samples were stored in isotonic thymol between sectioning and testing.

*Toothpaste treatment*

The 28 dentine samples were divided into 7 groups (n=4), to be treated with: 5 toothpastes (as above) with different sized NovaMin® particles (2, 4, 7, 10 and 15 $\mu$m), a NovaMin®-free control (0%), and an AS control

Samples were brushed for 5 consecutive days, twice a day, for 20 seconds. A soft manual toothbrush head was used on a reciprocating brushing rig [Phoenix Tribology, Kingsclere, UK], rotating in a 5 mm radius circular motion under 150 g of applied mass. 2 mg of paste per treatment was applied to the dentine surface, where relevant, with 0.5 ml of AS. Samples were stored in a 3°C fridge in 15 ml of AS between brushing events, which was changed for fresh each night. This was to prevent bacterial growth on the samples, as no preservative was included in the AS.

*Nanoindentation*

Each dentine disc was indented 24 times (n=96 per group) using a Berkovich indenter tip fitted to a NanoTest Vantage system [Micro Materials Ltd, Wrexham, UK]. A fixed load of 1.5 mN was applied and removed over 7.5 seconds each (0.2 mN/s), with a 60 second hold period at maximum load to allow for creep, and a further 60 second hold period at 10% load post-indentation to calculate thermal drift correction. Samples were fully immersed in a liquid cell containing AS, meaning indentation was done in the tissue's natural, hydrated state. Finally, indents were spaced 40 $\mu$m apart, to prevent overlapping interaction volumes underneath the indented regions.

The unloading curve of each indent was analysed by the NanoTest Platform Four software version 5040.80.00 [Micro Materials Ltd, Wrexham, UK] to provide the hardness and reduced modulus of each indent. Hardness, H, is calculated using Equation 6.1, where Pmax is the maximum indent load, and Ac is the area of contact between the indenter tip and sample surface. Reduced modulus, Er, is calculated using Equation 6.2, where S is the stiffness (taken as the gradient of the unloading curve at maximum load), and $\beta$ is a correction factor depending on the indenter tip geometry

(for a Berkovich indenter, this is 1.034).

$$H = \frac{P_{max}}{A_c} \tag{6.1}$$

$$E_r = \frac{\sqrt{\pi}}{2\beta} \frac{S}{\sqrt{A_c}} \tag{6.2}$$

*Glancing Angle X-Ray Diffraction*

Following nanoindentation, three samples from each group were left to fully air dry, before scanning with GA-XRD [Rigaku SmartLab, Tokyo, Japan]. Each sample was scanned at room temperature between 10 and 60 °2θ, in 0.02 °2θ steps. Scanning at a rate of 5 degrees per minute resulted in a total scan time of 10 minutes per sample. The angle of incidence was set at 1 degree from the surface. The three spectra per group were averaged for qualitative comparison between groups.

Further analysis focussed on the peak present at approximately 26 °2θ, corresponding to the (002) phase of HA. This is the second largest peak, however the largest, at approximately 32 °2θ, is comprised of multiple overlapping peaks corresponding to multiple phases of HA, meaning quantification may not be as accurate. Using graphical analysis software SigmaPlot V13.0, a four parameter Gaussian curve was fitted to the peak at 26 °2θ

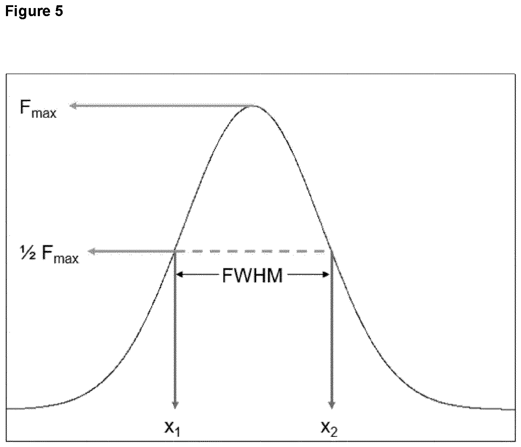

From this fitted curve, a FWHM was calculated, as shown in Figure 5.

This allowed for the average crystal size to be calculated for the layers using the Scherrer equation:

$$D = \frac{K\lambda}{\beta \cos\theta}$$

Where D is the crystallite size in nm, K is the Scherrer constant (a dimensionless shape factor commonly taken to be 0.9), $\lambda$ is the wavelength of the x-ray, $\beta$ is the FWHM and $\theta$ is the peak position.

*X-Ray Diffraction*

For this investigation, the reactive properties of the NovaMin® BAG alone were investigated. NovaMin® powder of the same five particle sizes (2 $\mu$m, 4 $\mu$m, 7 $\mu$m, 10 $\mu$m and 15 $\mu$m) was provided by Haleon [Weybridge, UK]. The powders were reacted in a ratio of 225 mg to 150 ml of AS, as used in literature, for 6 different times: 1, 4, 8, 12, 24 and 36 hours. After each allotted time, the powder was removed from the AS, filtered using 2.5 $\mu$m retention filter paper [Cytiva Whatman™, Massachusetts, USA], and air-dried for at least 2 hours on the filter paper. Drying was then finished in a 60°C oven for 6 minutes, and powders stored in a desiccator until testing.

The resulting powder was scanned using XRD [D2 Phaser, Bruker, Massachusetts, USA] to detect the presence of crystalline apatite. The powder was scanned at room temperature, between 15 and 60 °$2\theta$, in 1308 steps. A time per step of 2 seconds was used, resulting in a scan length of approximately 45 minutes per sample. The spectra were automatically normalised against a background scan by the accompanying software. Each particle size at 12, 24 and 36 hours was reacted and scanned in triplicate, whereas 1, 4 and 8 hours were generated once, owing to time constraints of the project, and the lack of quantification possible due to the highly amorphous nature of the powder still so early on. For comparison, a sample of fully crystalline HA powder was also scanned under identical conditions.

Evaluation of crystallite size was repeated using the Scherrer Equation 6.5. It should be noted that this only occurred on samples from 12, 24 and 36 hours, as SigmaPlot was unable to accurately fit a curve to the peaks at earlier stages, due to it either not being present, or not clearly formed.

**Results**

*Nanoindentation*

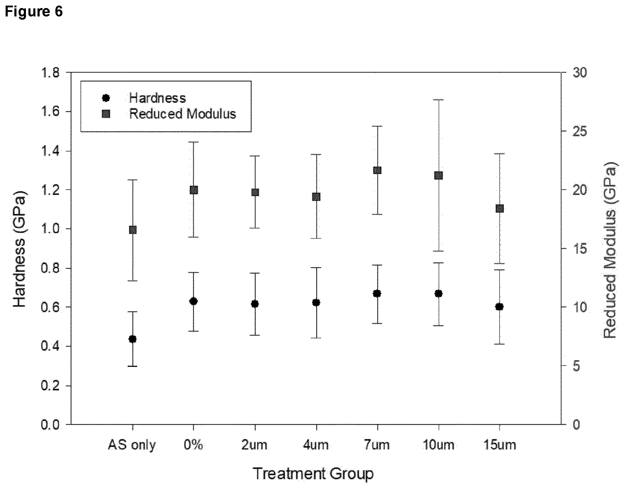

The results for both hardness and reduced modulus of the HA-like surface layer are presented in Table 2, and graphically in Figure 6.

The AS control had a significantly lower average hardness value ($p < 0.001$) than all other groups, however there was no significant difference between any BAG particle size or the NovaMin®-free control. The AS control also had a significantly lower reduced modulus ($p < 0.05$) than all other groups except the 15 $\mu$m BAG formulation. A statistical significance was also found between the 7 and 15 $\mu$m formulations ($p = 0.013$), however the 15 $\mu$m particle size had one of the smallest numbers of usable indents due to poor sample quality and surface topography ($n = 30$), and so both of these statistical findings are thought to be artefacts of this complication. No statistical significance was observed between the remaining particle sizes or 0% control. This lack of statistical significance between particle sizes on both the hardness and reduced modulus results stems from the large standard deviations evident on Figure 5. The same trend was observed with respect to the maximum indentation depth, with all treated NovaMin groups having shallower indents than either control. The standard deviations of these groups were also smaller, indicating more consistent results.

The microhardness results, despite being fewer in number, also showed large variations in the results seen. A similar order of magnitude was observed to the results presented in Table 6. 1, with all groups combined averaging a hardness of 0.67 GPa $\pm$ 0.1 GPa, except the 15 $\mu$m NovaMin® group. This sample was considerably softer (0.24 GPa $\pm$ 0.01 GPa) and is likely a problem regarding the dentine substrate. This would not have been detected as clearly in the nanoindentation testing, as only the surface layer was evaluated, however may explain the statistically lower average reduced modulus discussed previously. There was no trend observed, and as only one sample per group was assessed (the others had been dehydrated for GA-XRD by this time), the potential for inter-sample variation to affect the results could not be ruled out.

*Table 2 (Average Hardness, Reduced Modulus and Maximum Indentation Depth results ($\pm$ 1 Standard Deviation))*

| Treatment Group | Hardness (GPa) | Reduced Modulus (GPa) | Max Depth (nm) |
| --- | --- | --- | --- |
| AS only 0% (NovaMin®-free) | 0.436 (0.14) | 16.566 (4.30) | 384 (109) |
|  | 0.628 (0.15) | 20.002 (4.06) | 373 (138) |
| 2 $\mu$m NovaMin® | 0.615 (0.16) | 19.793 (3.08) | 302 (38) |

(continued)

| Treatment Group | Hardness (GPa) | Reduced Modulus (GPa) | Max Depth (nm) |
|---|---|---|---|
| 4 $\mu$m NovaMin® | 0.623 (0.18) | 19.424 (3.57) | 313 (68) |
| 7 $\mu$m NovaMin® | 0.668 (0.15) | 21.663 (3.77) | 290 (54) |
| 10 $\mu$m NovaMin® | 0.667 (0.16) | 21.231 (6.44) | 298 (61) |
| 15 $\mu$m NovaMin® | 0.601 (0.19) | 18.400 (4.69) | 292 (46) |

*Glancing Angle X-Ray Diffraction*

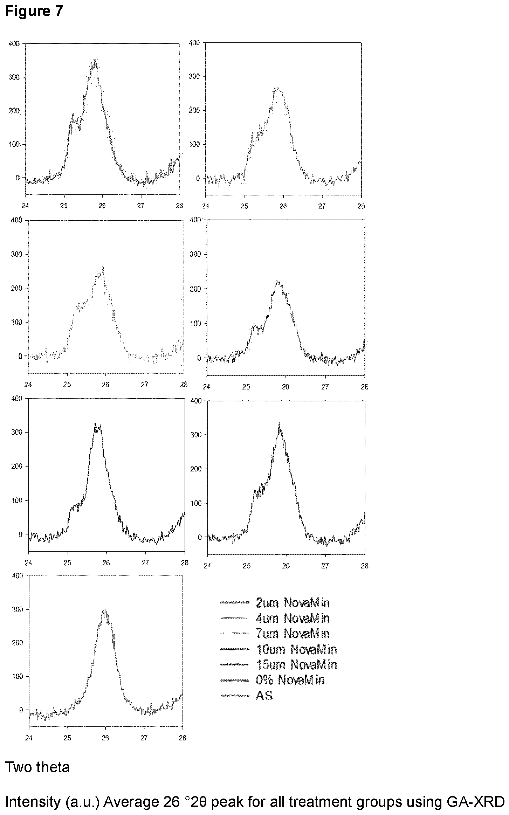

Figure 7 shows the average 26 °2θ peak for all 7 groups. All BAG groups plus the NovaMin®-free control show signs of peak splitting, with the formation of a shoulder on the left side of the usual peak. This shoulder corresponds to Titanium Dioxide (TiO2), which is another ingredient included within the toothpaste. This explains the lack of this peak in the AS-only control. The intensity of the secondary peak appears to decrease with increasing particle size, indicating that it is more present in the HA-like material produced by the smaller particles. However interestingly, the 0% NovaMin® control also shows a high intensity, similar to that of the 4 $\mu$m particle size. For the purposes of the Gaussian curve fitting required to find FWHM, this shoulder was ignored, and the curve fitted to the main peak only. All fits had an r-squared value of 93.73% $\pm$ 4.6%. The average crystallite size was found to be 14.28 $\pm$ 2.3 nm for all NovaMin® groups, 14.15 $\pm$ 0.9 nm for the 0% control, and 13.93 $\pm$ 1.9 nm for the AS control. There was no significant difference found (p = 0.722). The intensity of the primary peak showed a similar trend - decreasing intensity with increasing particle size - with the exception of the 15 $\mu$m particle size. Both this, the 0% and the AS control all had intensities similar to the 2 and 4 $\mu$m BAG groups.

*X-Ray Diffraction*

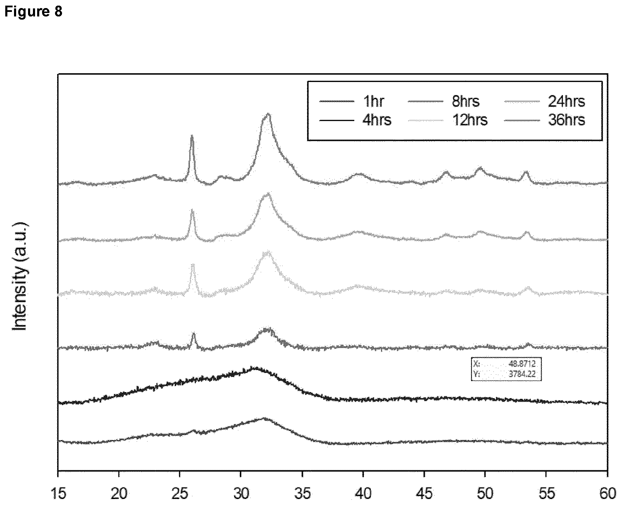

Figure 8 shows representative spectra from 1 to 36 hours of the 2 $\mu$m NovaMin® particle size.

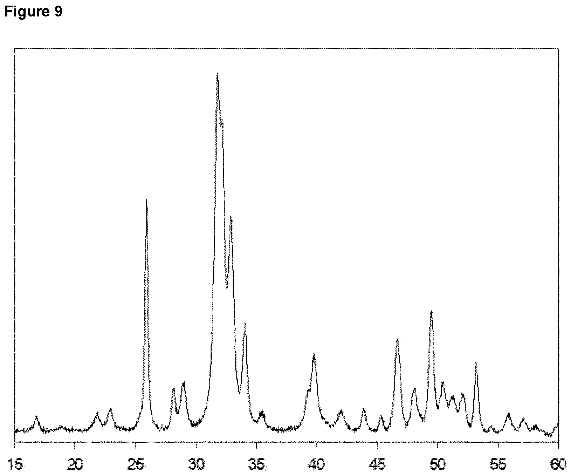

As the experimental time progresses, the peaks become clearer and sharper, with the two main peaks at approximately 26 °2θ and 32 °2θ becoming apparent after only 8 hours. The increasing intensity and sharpness of the peaks indicates a transition from the broad amorphous trace to an organised, crystalline solid once the BAG powder has had sufficient time to react with AS and form apatite. However, comparing this with the spectrum of pure HA powder scanned under identical conditions, shown in Figure 9, and it is clear that the filtered powder is still considerably amorphous. The peaks in Figure 8 are broader than the sharper peaks seen in Figure 9, which indicate a highly organised and fully crystalline structure.

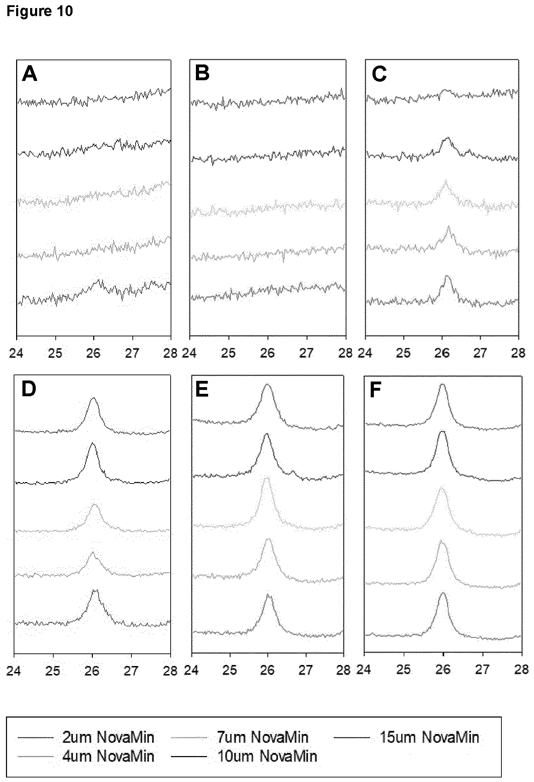

Comparing between individual particle sizes, Figure 10 A - F show the results from each timepoint respectively for all 5 particle sizes, with focus on the 26 °2θ peak. Images A and B (1 and 4 hours) show no significant trace of a peak, indicating that the apatite has not had sufficient time to react and crystallise. However, by 8 hours (Figure 10 C), the (002) plane peak is beginning to form. This peak appears sharper and more intense in the lower particle sizes, indicating greater reactivity, as more apatite has formed in the same amount of time. By 12 hours, this peak is sharper and more intense for all particle sizes, with less of an obvious trend, however the smallest particle size of 2 $\mu$m is still visually one of the most intense; up to 30% greater than 4 and 7 $\mu$m, and within 12% of the larger particles of 10 and 15 $\mu$m. However, there was no statistical difference between intensities at 12 hours (p = 0.425). Finally, after 24 and 36 hours (Figure 10 E and F respectively), the peaks are well formed, sharp and indicate a crystalline apatite structure. There is no significant difference or visual trend in intensity of the peaks when comparing between particle sizes (p = 0.613 for 24 hours, p = 0.831 for 36 hours).

Average crystallite sizes varied between 17 and 25 nm across all groups in the three latter time points, with no observable trend resulting from changing the particle size. There was no significant difference between crystallite size at 12 and 24 hours (p = 0.929 and 0.700 respectively), however at 36 hours, the 7 $\mu$m BAG powder did produce statistically smaller crystallites.

*Discussion*

Overall, the particle size of the NovaMin® glass included in the full formulation appeared to have no significant effect on the mechanical or crystalline properties of the HA-like layer once it was formed on the surface of the dentine. After interrogation with nanoindentation, there was no significant difference in either the hardness or the reduced modulus of the HA-like layer, shown in Figure 5. GA-XRD also showed no significant difference in the size of crystallites within this HA-like layer after 5 days. The crystallite size results of the powder XRD showed no significant difference after 12 or 24 hours. Comparisons of the 26 °2θ peaks at the earlier timepoints (<12 hours) however showed the smaller particles form apatite material faster and are therefore more reactive.

The work has showed nanoindentation as a valuable technique for interrogating the mechanical properties of treated dentine samples. The ability to perform tests while samples are fully hydrated and therefore in their natural physiological state can improve the accuracy of the data and the applicability to real-life scenarios. Also, the ability to limit the depth of the indents to only investigate the surface HA-like layers is an improvement over previous techniques that penetrate much deeper into the dentine substrate. This reduces the likelihood of producing a composite response of both substrate and surface layer, which may introduce uncertainty, masking trends in results. As shown in Table 2, the average indent depth of a NovaMin treated sample was approximately 300nm. Using the 10% rule, which assumes the maximum penetrated depth represents approximately 10% of the interacted volume, this would result in an interaction volume of 3 $\mu$m. Previous studies have recorded the effects of several BAGs, including NovaMin, to be detectable up to 30 $\mu$m into the tissue, meaning these groups would have been only interacting with the treated surface. This would also explain the smaller standard deviations observed on these groups, indicating a more consistent surface. Conversely, the two control groups penetrated much deeper, and also had much more inconsistent results. Observing the results of the SBF SEM imaging presented in Chapter 5, there was not a consistent layer after 2 days on either control sample, so it is assumed that after 5 days, this would also be the case due to the lack of an active ingredient to react. Therefore, these results of hardness and reduced modulus of the control samples are likely a composite response of a smear layer (if present), and the underlying dentine matrix. It is believed that this was the case with the small number microhardness tests performed, resulting in low statistical power and no trend in results. These microhardness tests had also penetrated deep enough to also measure more of the underlying dentine substrate. As discussed, the HA-like surface layer and Intertubular Dentine both had similar grey values, meaning they had a similar density. This may explain why the microhardness tests were very similar to the nanoindentation results, which tested only the surface layer, as microhardness testing would have been unable to detect smaller changes in material properties.

The samples of bovine dentine used for the nanoindentation tests varied highly in quality. Some showed signs of acid damage, likely from the removal of the smear layer by the manufacturer. This issue was not found until a significant change in mechanical properties was noticed during indentation, and thus the samples had to be discounted from further testing. Acid damage is known to affect the organic component of dentine more readily than the crystalline, resulting in a loss or denaturation of the collagen network. As this collagen is an important part of the NovaMin reaction into apatite, acid damage to the samples would have inhibited these reactions, resulting in a nonrepresentative apatite layer. Hence, samples with this damage were discounted. It was ensured that a minimum of 2 usable samples remained per group, to reduce the risk of inter-sample variation. However, when also discounting "false contact" indents, no group was able to achieve more than 60 successful indents out of a possible 96. Also, as a third party supplied these samples, there was no way to control for the depth of dentine used, relative to the whole tooth. Dentine closer to the pulp is less mineralised, and therefore may respond differently to abrasive treatments, such as brushing, or mechanical testing.

The lack of difference in crystallite size as measured on the GA-XRD data after 5 days correlates with the findings from the powder-based XRD data. There was also no significant difference found in the crystallite size measured in the reacted Bioactive Glass powders after 12 or 24 hours. Only the 7 $\mu$m produced significantly smaller crystallites after 36 hours, there was no significant difference between the others ($p > 0.05$). While statistically significant, a change of ~3 nm, when compared to the average crystallite of HA existing naturally within dentine (60-70 nm in length, 20-30 nm in width), was not deemed substantial enough for further interrogation. It is possible that this significance was an artefact of low sample numbers (n = 3), or a difference in the dentine substrate, e.g. less mineralised dentine from a different part of the tooth. Despite using a very shallow glancing angle of 1°, it is possible some of the trace is detecting hydroxyapatite from within the dentine, which may have had an influence, and caused a statistical, but practically unsubstantial, difference.

On the other hand, there was a comparative difference found in the earlier timepoints. The smallest particle sizes were found to form a sharper and higher intensity peak at 26 °2$\theta$ sooner, indicating a greater presence of crystalline apatite material. Overall, this indicates a greater short-term reactivity ($\leq$ 12 hours) of the smallest Bioactive Glass particle size of 2 $\mu$m. The larger particle sizes still have the same apatite forming capabilities, as shown by the similar peak intensities at later timepoints, however they are clearly not as able to form these as quickly. The spectra still showed high amorphous content in comparison to the purely crystalline HA presented in Figure 9. Nonetheless, NovaMin® is known to bind to the dentine surface and continue the mineralisation reactions after the initial supply of materials. Also, these powder XRD comparisons are based on one amount of powder continuously reacting for up to 36 hours. However, in reality, both the Bioactive Glass and saliva would be refreshed and restored over time with regular brushing and saliva regeneration, providing more active ingredient and more mineralisation potential. The use of the Scherrer equation was effective at approximating crystallite size in both the powder and glancing angle XRD work presented here, with r-squared values indicating high accuracy of the Gaussian fits. This technique is commonly used in the literature, and these results are a similar order of magnitude to those previously reported. The appearance of measurable crystallites was also noted in both control groups, as well as in all NovaMin® groups. This was not unexpected, as the use of a dentine substrate is likely to produce these peaks due to the tissue's own apatite content (approximately 45% of intertubular dentine). The similarities between the GA-XRD traces of the NovaMinO-treated and control dentine samples indicate that the surface layers formed are indeed structurally and chemically similar to that of HA (Figure 7). This is also confirmed by comparing the resulting peaks from the powder XRD to that of pure HA (Figure 8 vs Figure 9).

*Figures*

Figure 1 shows the Tubule Occlusion test method.
Figures 2A & 2B show Tubule Occlusion: Depth Measurement.
Figure 3 shows the average depth of occlusion for each group after 24 and 48 hours.
Figure 4 shows Tubule Occlusion Depth Results.
Figure 5 shows how a FWHM was calculated.
Figure 6 shows hardness and reduced modulus of the HA-like surface layer.
Figure 7 shows Average 26 °2θ peak for all treatment groups using GA-XRD.
Figure 8 shows X-Ray Diffraction representative spectra from 1 to 36 hours of the 2 $\mu$m NovaMin® particle size.
Figure 9 shows an X-Ray Diffraction spectrum of pure HA powder.
Figure 10 shows (shows 26 °2θ peak from XRD after A) 1 hour, B) 4 hours, C) 8 hours, D) 12 hours, E) 24 hours and F) 36 hours.

**Claims**

1. A dentifrice composition comprising a tubule blocking agent, wherein the tubule blocking agent comprises a bioactive glass, and wherein the tubule blocking agent has a particle size less than 10 $\mu$m.

2. The dentifrice composition according to claim 1 wherein the bioactive glass has a composition comprising 45 wt % $SiO_2$, 24.5 wt % CaO, 24.5 wt % $Na_2O$, and 6.0 wt % $P_2O_{5)}$

3. The dentifrice composition according to claim 1 or claim 2, wherein the tubule blocking agent has a particle size less than about 9 $\mu$m, more preferably less than about 8 $\mu$m, more preferably less than about than 7 $\mu$m, more preferably less than about 6 $\mu$m, more preferably less than about 5 $\mu$m, more preferably less than about 4 $\mu$m, more preferably less than about 3 $\mu$m, more preferably less than about 2.5 $\mu$m, more preferably less than about 2.25 $\mu$m, more preferably less than about 2.1 $\mu$m, more preferably less than about 2 $\mu$m.

4. The dentifrice composition according to claim 1 or claim 2, wherein the tubule blocking agent has a particle size in the range of about 0.01 to about 10 $\mu$m, more preferably in the range of about 0.01 to about 9 $\mu$m, more preferably in the range of about 0.01 to about 8 $\mu$m, more preferably in the range of about 0.01 to about 7 $\mu$m, more preferably in the range of about 0.01 to about 6 $\mu$m, more preferably in the range of about 0.01 to about 5 $\mu$m, more preferably in the range of about 0.01 to about 4 $\mu$m, more preferably in the range of about 0.01 to about 3 $\mu$m, more preferably in the range of about 0.01 to about 2.5 $\mu$m, more preferably in the range of about 0.01 to about 2.25 $\mu$m. more preferably in the range of about 0.01 to about 2.1 $\mu$m, more preferably in the range of about 0.01 to about 2 $\mu$m.

5. The dentifrice composition according to claim 1 or claim 2, wherein the tubule blocking agent has an average particle size in the range of about 0.01 to about 10 $\mu$m, more preferably in the range of about 0.01 to about 9 $\mu$m, more preferably in the range of about 0.01 to about 8 $\mu$m, more preferably in the range of about 0.01 to about 7 $\mu$m, more preferably in the range of about 0.01 to about 6 $\mu$m, more preferably in the range of about 0.01 to about 5 $\mu$m, more preferably in the range of about 0.01 to about 4 $\mu$m, more preferably in the range of about 0.01 to about 3 $\mu$m, more preferably in the range of about 0.01 to about 2.5 $\mu$m, more preferably in the range of about 0.01 to about 2.25 $\mu$m, more preferably in the range of about 0.01 to about 2.1 $\mu$m, more preferably in the range of about 0.01 to about 2 $\mu$m. In each of these ranges the lower amount (about 0.01 $\mu$m) may be replaced by about 0.1 $\mu$m, about 0.2 $\mu$m, about 0.3 $\mu$m, about 0.4 $\mu$m, about 0.5 $\mu$m, about 0.6 $\mu$m, about 0.7 $\mu$m, about 0.8 $\mu$m, about 0.9 $\mu$m, about 1.0 $\mu$m, 1.1 $\mu$m, about 1.2 $\mu$m, about 1.3 $\mu$m, about 1.4 $\mu$m, about 1.5 $\mu$m, about 1.6 $\mu$m, about 1.7 $\mu$m, about 1.8 $\mu$m, or about 1.9 $\mu$m (wherein the tubule blocking agent has a particle size less than 10 $\mu$m).

6. The dentifrice composition according to claim 4 or 5, wherein in each of these ranges the lower amount (about 0.01 $\mu$m) may be replaced by about 0.1 $\mu$m, about 0.2 $\mu$m, about 0.3 $\mu$m, about 0.4 $\mu$m, about 0.5 $\mu$m, about 0.6 $\mu$m, about 0.7 $\mu$m, about 0.8 $\mu$m, about 0.9 $\mu$m, about 1.0 $\mu$m, 1.1 $\mu$m, about 1.2 $\mu$m, about 1.3 $\mu$m, about 1.4 $\mu$m, about 1.5 $\mu$m, about 1.6 $\mu$m, about 1.7 $\mu$m, about 1.8 $\mu$m, or about 1.9 $\mu$m.

7. The dentifrice composition according to any of the preceding claims, wherein the tubule blocking agent is present in an amount of 0.1 to 10 percent by weight of the composition.

8.  The dentifrice composition according to any of the preceding claims, wherein the composition comprises a single phase non-aqueous composition comprising at least one solvent, and wherein the at least one solvent comprises up to 85 percent by weight of the final composition.

9.  The dentifrice composition according to claim 8, wherein the at least one solvent comprises glycerol, polyethylene glycol (PEG) or a mixture thereof.

10. The dentifrice composition according to any of the preceding claims, wherein an abrasive is present in an amount up to 10 percent by weight of the total composition.

11. The dentifrice composition according to claim 10, wherein the abrasive is selected from the group comprising amorphous, gelled, precipitated or fumed silica, zinc orthophosphate, sodium bicarbonate (baking soda), plastic particles, alumina, hydrated alumina, calcium carbonate, calcium pyrophosphate, insoluble metaphosphates or mixtures thereof.

12. The dentifrice composition according to any of the preceding claims, wherein the composition comprises a source of fluoride.

13. The dentifrice composition according to claim 12, wherein source of fluoride is selected from the group comprising stannous fluoride, sodium fluoride, sodium monofluorophosphate, potassium fluoride, ammonium fluoride, bis-(hydroxyethyl) amino-propyl-N-hydroxyethyloctadecylamine-dihydrofluoride and mixtures thereof. Preferably the fluoride required in the formula will be provided by stannous fluoride (SnF2) and / or sodium fluoride (NaF).

14. The dentifrice composition according to any of the preceding claims, wherein the composition comprises a surfactant.

15. The dentifrice use of the composition according to any of claims 1 to 14 in combination with a toothbrush to clean teeth.

**Figure 1**

**Figures 2A & 2B**

**Figure 3**

**Figure 4**

**Figure 5**

**Figure 6**

## Figure 7

2um NovaMin
4um NovaMin
7um NovaMin
10um NovaMin
15um NovaMin
0% NovaMin
AS

Two theta

Intensity (a.u.) Average 26 °2θ peak for all treatment groups using GA-XRD

**Figure 8**

**Figure 9**

**Figure 10**

Europäisches Patentamt
European Patent Office
Office européen des brevets

**EUROPEAN SEARCH REPORT**

Application Number

EP 24 21 6016

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | "BioMin F: Who needs 5000 ppm or 1450 ppm for that matter?", BRITISH DENTAL JOURNAL : BDJ ; THE JOURNAL OF THE BRITISH DENTAL ASSOCIATION, NATURE PUBLISHING GROUP, BRITISH DENTAL ASSOCIATION, UK, vol. 232, no. 5, 11 March 2022 (2022-03-11), page 345, XP037713401, ISSN: 0007-0610, DOI: 10.1038/S41415-022-4082-4 [retrieved on 2022-03-11] | 1-6,12, 15 | INV. A61K8/02 A61K8/25 A61Q11/00 |
| Y | * column 2, paragraph third * * the whole document * | 1-15 | |
| X | DATABASE GNPD [Online] MINTEL; 5 July 2021 (2021-07-05), anonymous: "Relieve+Shield Toothpaste for Sensitive Teeth", XP093273113, Database accession no. 8833537 | 1-6, 12-15 | |
| Y | * ingredient list; the whole document * | 1-15 | TECHNICAL FIELDS SEARCHED (IPC) A61K A61Q |
| X | DATABASE MEDLINE [Online] US NATIONAL LIBRARY OF MEDICINE (NLM), BETHESDA, MD, US; June 2016 (2016-06), ZANG PENG ET AL: "A Randomized Clinical Trial Investigating the Effect of Particle Size of Calcium Sodium Phosphosilicate (CSPS) on the Efficacy of CSPS-containing Dentifrices for the Relief of Dentin Hypersensitivity.", XP002813292, Database accession no. NLM28390207 | 1-6,12, 15 | |
| Y | * abstract * | 1-15 | |

-/--

The present search report has been drawn up for all claims

| Place of search | Date of completion of the search | Examiner |
|---|---|---|
| Munich | 6 May 2025 | Briand, Benoit |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 

& : member of the same patent family, corresponding document

EPO FORM 1503 03.82 (P04C01)

page 1 of 2

Europäisches
Patentamt

European
Patent Office

Office européen
des brevets

# EUROPEAN SEARCH REPORT

**Application Number**

EP 24 21 6016

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | Mahmood Asad ET AL: "Abrasive wear of enamel by bioactive glass-based toothpastes", , 1 October 2014 (2014-10-01), XP093273142, Retrieved from the Internet: URL:https://d1wqtxts1xzle7.cloudfront.net/ 106133494/Abrasive_20wear_20of_20enamel_20 by_20bioactive_20glass-based_20toothpastes -libre.pdf?1696243676=&response-content-di sposition=inline;+filename=Abrasive_wear_o f_enamel_by_bioactive_gla.pdf&Expires=1745 843729&Signature=Ch7FU6aF3RcvTlcy2EEKUFxkz 2o0HchxkZq | 1-11,14, 15 | |
| Y | * page 1, column 1, paragraph 1 * * table 2 * ----- | 1-15 | |
| X | EP 3 668 604 B1 (UNILEVER GLOBAL IP LTD [GB]; UNILEVER IP HOLDINGS B V [NL]) 13 October 2021 (2021-10-13) | 1-7,14, 15 | |
| Y | * paragraphs [0042], [0043], [0053], [0061]; claim 1 * ----- | 1-15 | **TECHNICAL FIELDS SEARCHED (IPC)** |
| X | US 9 937 365 B2 (CHOPRA SUMAN K [US]; ZAIDEL LYNETTE [US] ET AL.) 10 April 2018 (2018-04-10) * claim 1; tables 1, 4 * * column 8, line 8 - column 9, line 26 * ----- | 1-15 | |
| A | Haleon Healthpartner Deutschland: "IM EXPERTENGESPRÄCH: NOVAMIN BEI SCHMERZEMPFINDLICHEN ZÄHNEN", , 5 August 2024 (2024-08-05), XP093273078, Retrieved from the Internet: URL:https://www.youtube.com/watch?v=OFWdph hARGo * the whole document * ----- | 1-15 | |

The present search report has been drawn up for all claims

| Place of search | Date of completion of the search | Examiner |
|---|---|---|
| Munich | 6 May 2025 | Briand, Benoit |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 24 21 6016

This annex lists the patent family members relating to the patent documents cited in the above-mentioned European search report.
The members are as contained in the European Patent Office EDP file on
The European Patent Office is in no way liable for these particulars which are merely given for the purpose of information.

06-05-2025

| Patent document cited in search report | | Publication date | Patent family member(s) | Publication date |
|---|---|---|---|---|
| EP 3668604 | B1 | 13-10-2021 | BR 112020001118 A2 | 21-07-2020 |
| | | | CN 110997072 A | 10-04-2020 |
| | | | EP 3668604 A1 | 24-06-2020 |
| | | | WO 2019034348 A1 | 21-02-2019 |
| US 9937365 | B2 | 10-04-2018 | AR 076178 A1 | 26-05-2011 |
| | | | AU 2010232505 A1 | 22-09-2011 |
| | | | BR PI1013182 A2 | 12-04-2016 |
| | | | CA 2756993 A1 | 07-10-2010 |
| | | | CN 102753135 A | 24-10-2012 |
| | | | CN 104983592 A | 21-10-2015 |
| | | | CO 6361890 A2 | 20-01-2012 |
| | | | EP 2413882 A2 | 08-02-2012 |
| | | | JP 5744838 B2 | 08-07-2015 |
| | | | JP 2012522800 A | 27-09-2012 |
| | | | MY 155413 A | 15-10-2015 |
| | | | RU 2011144099 A | 10-05-2013 |
| | | | RU 2014139014 A | 20-04-2016 |
| | | | SG 173881 A1 | 28-10-2011 |
| | | | TW 201102097 A | 16-01-2011 |
| | | | US 2012021031 A1 | 26-01-2012 |
| | | | WO 2010115039 A2 | 07-10-2010 |
| | | | ZA 201106823 B | 26-03-2014 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 0215809 A, Block

- EP 18782931

**Non-patent literature cited in the description**

- *CHEMICAL ABSTRACTS*, 7631-86-9